# EUROPÄISCHE PATENTSCHRIFT

(11) **EP 1 030 847 B1**
(45) Veröffentlichungstag und Bekanntmachung des Hinweises auf die Patenterteilung: **22.03.2006**
(21) Anmeldenummer: 98947550.4
(22) Anmeldetag: 25.09.1998
(51) Int. Cl.: C07D 249/12

(54) **VERFAHREN ZUR HERSTELLUNG VON TRIAZOLINTHION-DERIVATEN**
METHOD FOR PRODUCING TRIAZOLINTHION DERIVATIVES
PROCEDE DE PREPARATION DE DERIVES DE THIONE DE TRIAZOLINE

(30) Priorität: 08.10.1997 DE 19744402; 01.09.1998 DE 19839688
(43) Veröffentlichungstag der Anmeldung: 30.08.2000
(73) Patentinhaber: Bayer CropScience AG, 40789 Monheim (DE)
(72) Erfinder: JAUTELAT, Manfred, D-51399 Burscheid (DE); ERDMAN, David, D-51061 Köln (DE)
(86) Internationale Anmeldenummer: PCT/EP1998/006112
(87) Internationale Veröffentlichungsnummer: WO 1999/018087

(56) Entgegenhaltungen:
- WO-A-96/16048
- DE-A- 4 030 039
- I. ARAI: BULLETIN OF THE CHEMICAL SOCIETY OF JAPAN, Bd. 46, Nr. 7, 1973, Seiten 2215-8, XP002087557 in der Anmeldung erwähnt

## Beschreibung

Die vorliegende Erfindung betrifft ein neues Verfahren zur Herstellung von Triazolinthion-Derivaten, die als Wirkstoffe mit mikrobiziden, insbesondere fungiziden Eigenschaften bekannt sind.

Es ist bereits bekannt, daß sich Triazolinthion-Derivate herstellen lassen, indem man die entsprechenden Triazol-Derivate entweder nacheinander mit starken Basen und Schwefel umsetzt und dann hydrolysiert oder direkt mit Schwefel bei hohen Temperaturen umsetzt und dann mit Wasser behandelt (vgl. WO-A 96-16 048). Nachteilig an diesen Verfahren ist aber, daß die gewünschten Produkte nur in relativ niedrigen Ausbeuten anfallen oder daß Umsetzungsbedingungen erforderlich sind, die in technischem Maßstab nur extrem schwierig einzuhalten sind.

Weiterhin wurde schon beschrieben, daß sich bestimmte, in 3-Position substituierte 1,2,4-Triazolin-5-thione dadurch herstellen lassen, daß man N-Chlorthioformyl-N-(1-chloralk-1-en)-amine mit Carbonylhydrazin-Derivaten umsetzt (vgl. DE-A 197 01 032, DE-A 196 01 189 und EP-A 0 784 053). Die Synthese von entsprechenden Substanzen, die keinen Substituenten in der 3-Position enthalten, wird jedoch nicht erwähnt.

Ferner geht aus Bull. Chem. Soc. Japan 46, 2215 (1973) hervor, daß sich in 3-Position substituierte Triazolinthione synthetisieren lassen, indem man Phenylhydrazin mit Natriumthiocyanat und Ketonen oder Aldehyden in Gegenwart von Salzsäure umsetzt und die dabei anfallenden, in 3-Position substituierten Triazolidinthione mit oxidierenden Reagenzien behandelt. Beeinträchtigend an diesem Verfahren ist, daß sehr lange Reaktionszeiten erforderlich sind und keine in 3-Stellung unsubstituierten Triazolinthione auf diese Weise zugänglich sind.

Schließlich ist auch schon bekannt, daß man 2-(1-Chlor-cycloprop-1-yl)-1-(2-chlorphenyl)-3-(1,2,4-triazol-1-yl)-propan-2-ol erhält, wenn man [1-(2-Chlor-phenyl)-2-(1-chlor-cycloprop-1-yl)-2-hydroxy]-propyl-hydrazin mit Formamidinacetat umsetzt (vgl. DE-A 40 30 039). Thiono-Derivate von Triazolen sind nach dieser Methode allerdings nicht zugänglich.

Es wurde nun gefunden, daß sich Triazolinthion-Derivate der Formel 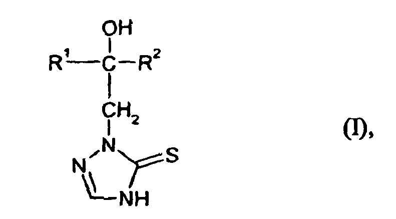
in welcher
- R¹ und R²: gleich oder verschieden sind geradkettiges oder verzweigtes Alkyl mit 1 bis 6 Kohlenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Alkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil unt 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,

herstellen lassen, indem man
a) in einer ersten Stufe Hydrazin-Derivate der Formel 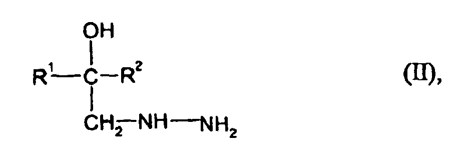 in welcher
   - R¹ und R²: die oben angegebenen Bedeutungen haben,
   mit Formaldehyd und Thiocyanat der Formel

   X-SCN (III),

   in welcher
   - X: für Natrium, Kalium oder Ammonium steht,

   in Gegenwart eines Verdünnungsmittels und gegebenenfalls in Gegenwart einer Säure umsetzt und
b) die entstandenen Triazolidinthione der Formel 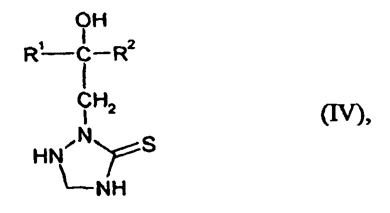
   in welcher
   - R¹ und R²: die oben angegebenen Bedeutungen haben,

   in einer zweiten Stufe
   entweder
   α) mit Oxidationsmitteln gegebenenfalls in Gegenwart eines Katalysators und in Gegenwart eines Verdünnungsmittels umsetzt
      oder
   β) mit Ameisensäure der Formel

      HCOOH (V)

   umsetzt.

Es ist als ausgesprochen überraschend zu bezeichnen, daß sich Triazolinthion-Derivate der Formel (I) nach dem erfindungsgemäßen Verfahren in wesentlich höheren Ausbeuten bzw. unter erheblich einfacheren Bedingungen herstellen lassen als nach den bisher bekannten Methoden.

Das erfindungsgemäße Verfahren zeichnet sich durch eine Reihe von Vorteilen aus. So ermöglicht es, wie schon erwähnt, die Synthese von Triazolinthion-Derivaten der Formel (I) in hoher Ausbeute. Günstig ist auch, daß die benötigten Ausgangsstoffe und Reaktionskomponenten einfach herstellbar und auch in größeren Mengen verfügbar sind. Ein weiterer Vorteil besteht schließlich darin, daß die Durchführung der einzelnen Reaktionsschritte und die Isolierung der Reaktionsprodukte keinerlei Schwierigkeiten bereitet.

Verwendet man [1-(2-Chlor-phenyl)-2-(1-chlor-cycloprop-1-yl)-2-hydroxy]-propyl-1-hydrazin als Ausgangssubstanz und setzt diese in der ersten Stufe mit Paraformaldehyd und Ammoniumthiocyanat um und läßt das dabei anfallende Produkt in der zweiten Stufe mit Sauerstoff in Gegenwart von Kaliumhydroxid und Schwefel reagieren, so kann der Verlauf des erfindungsgemäßen Verfahrens durch das folgende Formelschema veranschaulicht werden.

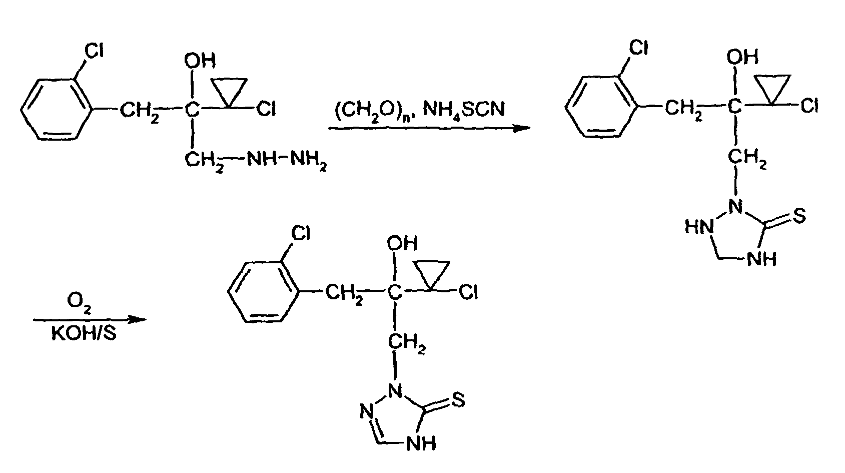

Die bei der Durchführung des erfindungsgemäßen Verfahrens als Ausgangsstoffe benötigten Hydrazin-Derivate sind durch die Formel (II) allgemein definiert.

Bevorzugt einsetzbar sind Hydrazin-Derivate der Formel (II), in welcher
- R¹: für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoxirninoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl, und
- R²: für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.

Besonders bevorzugt einsetzbar sind Hydrazin-Derivate der Formel (II), in welcher
- R¹: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-Cyclopropyl, Cyclopropyl, 1-Methylcyclopentyl oder 1-Ethyl-cyclopentyl steht,
oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordi- fluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluonnethoxy, Difluonnethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyallcinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl, und
- R²: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-cyclopropyl, Cyclopropyl, 1-Methylcyclopentyl oder 1-Ethyl-cyclopentyl steht,
oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximinoethyl, Nitro und/oder Cyano, Formyl, Dimethoxymethyl, Acetyl und/oder Propionyl.

Die Hydrazin-Derivate der Formel (II) sind bekannt oder lassen sich nach prinzipiell bekannten Verfahren herstellen (vgl. DE-A 40 30 039).

So erhält man Hydrazin-Derivate der Formel (II), indem man 1-Chlor-2-hydroxyethan-Derivate der Formel 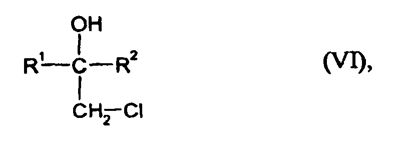
in welcher
- R¹ und R²: die oben angegebenen Bedeutungen haben,
in Gegenwart eines Säurebindemittels, wie Kalium-tert.-butylat, Natriummethylat oder Kaliumcarbonat, und in Gegenwart eines Verdünnungsmittels, wie Dimethylformamid, Methanol, n-Butanol, Tetrahydrofuran oder Methyl-tert.-butylether, bei Temperaturen zwischen 20°C und 60°C umsetzt und die dabei entstehenden Oxiran-Derivate der Formel 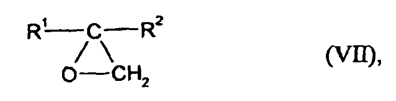
in welcher
- R¹ und R²: die oben angegebenen Bedeutungen haben,
mit Hydrazin-hydrat gegebenenfalls in Gegenwart eines Verdünnungsmittels, wie Methanol, n-Butanol, Tetrahydrofuran, Methyl-tert.-butylether, Dioxan oder Nitrile, wie Acetonitril, bei Temperaturen zwischen 30°C und 120°C umsetzt.

Besonders bevorzugt ist die Umsetzung der 1-Chlor-2-hydroxy-ethan-Derivate der Formel (VI) mit überschüssigem Hydrazin-hydrat, das hierbei sowohl als Reaktand als auch als Base und Verdünnungsmittel fungiert.

Die 1-Chlor-2-hydroxy-ethan-Derivate der Formel (VI) sind bekannt oder lassen sich nach prinzipiell bekannten Verfahren herstellen (vgl. DE-A 40 30 039 und EP-A 0 297 345).

Der bei der Durchführung der ersten Stufe des erfindungsgemäßen Verfahrens als Reaktionskomponente benötigte Formaldehyd kann als Paraformaldehyd, als gasförmiger Formaldehyd oder auch als Formalin-Lösung (= wäßrige FormaldehydLösung) eingesetzt werden.

Weiterhin werden bei der Durchführung der ersten Stufe des erfindungsgemäßen Verfahrens Thiocyanate der Formel (III) als Reaktionskomponenten eingesetzt. Die Thiocyanate der Formel (III) sind bekannt.

Als Verdünnungsmittel kommen bei der Durchführung der ersten Stufe des erfindungsgemäßen Verfahrens alle für derartige Umsetzungen üblichen, inerten organischen Solventien in Betracht. Vorzugsweise verwendbar sind Alkohole, wie n-Butanol und tert.-Butanol, ferner Ether, wie Dioxan und Methyl-tert.-butylether, außerdem Nitrile, wie Acetonitril, und Kohlenwasserstoffe, wie Toluol oder Chlorbenzol, jeweils gegebenenfalls im Gemisch mit Wasser.

Als Säuren können bei der Durchführung der ersten Stufe des erfindungsgemäßen Verfahrens alle üblichen anorganischen und organischen Säuren eingesetzt werden. Vorzugsweise verwendbar sind Salzsäure, Schwefelsäure, p-Toluolsulfonsäure und auch Natriumhydrogensulfat in Gegenwart von Wasser.

Sowohl bei der Durchführung der ersten als auch der zweiten Stufe des erfindungsgemäßen Verfahrens arbeitet man im allgemeinen unter Atmosphärendruck. Es ist aber auch möglich, unter erhöhtem Druck oder, sofern keine gasförmigen Komponenten an der Reaktion beteiligt sind, auch unter vermindertem Druck zu arbeiten.

Bei der Durchführung der ersten Stufe des erfindungsgemäßen Verfahrens setzt man auf 1 mol an Hydrazin-Derivat der Formel (II) im allgemeinen 1 bis 2 mol an Formaldehyd sowie 1 bis 2 mol an Thiocyanat der Formel (III) und gegebenenfalls eine äquivalente Menge an Säure bzw. Natriumhydrogensulfat und Wasser ein, wobei Wasser auch im Überschuß vorhanden sein kann. Die Aufarbeitung erfolgt nach üblichen Methoden. Arbeitet man in Abwesenheit von Wasser, so geht man im allgemeinen so vor, daß man das Reaktionsgemisch, gegebenenfalls nach vorherigem Verdünnen mit einem mit Wasser wenig mischbaren organischen Lösungsmittel, mit wäßrig-alkalischer Lösung wäscht, die organische Phase trocknet und einengt.

Arbeitet man in Gegenwart von Wasser und in Gegenwart von Natriumhydrogensulfat, so geht man im allgemeinen so vor, daß man das Reaktionsgemisch mit einem mit Wasser wenig mischbaren organischen Lösungsmittel verdünnt, filtriert und unter vermindertem Druck einengt. Gegebenenfalls danach noch vorhandene Verunreinigungen lassen sich nach üblichen Methoden, wie Umkristallisation oder Chromatographie, entfernen.

Die bei der Durchführung der zweiten Stufe des erfindungsgemäßen Verfahrens als Ausgangsstoffe benötigten Triazolidinthione sind durch die Formel (IV) allgemein definiert. In dieser Formel haben R¹ und R² vorzugsweise diejenigen Bedeutungen, die bereits im Zusammenhang mit der Beschreibung der Hydrazin-Derivate der Formel (II) vorzugsweise für diese Reste genannt wurden.

Die Triazolidinthione der Formel (IV) sind bisher noch nicht bekannt. Sie lassen sich durch die Umsetzung in der ersten Stufe des erfindungsgemäßen Verfahrens herstellen.

Als Oxidationsmittel kommen bei der Durchführung der zweiten Stufe des erfindungsgemäßen Verfahrens nach Variante (α) vorzugsweise Sauerstoff, Schwefel und Kaliumsuperoxid in Betracht. Setzt man als Oxidationsmittel Sauerstoff ein, so wird dieser vorzugsweise in Form von Luft zugeführt.

Als Katalysator kommt bei der Durchführung der zweiten Stufe des erfindungsgemäßen Verfahrens nach Variante (α) vorzugsweise ein Gemisch aus Kaliumhydroxid und Schwefel-Pulver in Frage. Dieser Katalysator wird vorzugsweise dann eingesetzt, wenn man Sauerstoff als Oxidationsmittel verwendet.

Als Verdünnungsmittel kommen bei der Durchführung der zweiten Stufe des erfindungsgemäßen Verfahrens nach Variante (α) alle für derartige Umsetzungen üblichen, inerten organischen Solventien in Betracht. Vorzugsweise verwendbar sind aliphatische, cycloaliphatische und aromatische Kohlenwasserstoffe, wie Pentan, Hexan, Cyclohexan, Benzol, Toluol und Xylol, ferner Ether, wie Methyl-tert.-butylether, und außerdem Ester, wie n-Butylacetat.

Die Reaktionstemperaturen können bei der Durchführung der zweiten Stufe des erfindungsgemäßen Verfahrens nach der Variante (α) innerhalb eines größeren Bereiches variiert werden. Im allgemeinen arbeitet man bei Temperaturen zwischen 0°C und 120°C. Setzt man Sauerstoff als Oxidationsmittel ein, so arbeitet man vorzugsweise bei Temperaturen zwischen 20°C und 80°C. Setzt man Schwefel als Oxidationsmittel ein, so arbeitet man vorzugsweise bei Temperaturen zwischen 50°C und 110°C. Setzt man Kaliumsuperoxid als Oxidationsmittel ein, so arbeitet man vorzugsweise bei Temperaturen zwischen 20°C und 80°C.

Bei der Durchführung der zweiten Stufe des erfindungsgemäßen Verfahrens nach Variante (α) setzt man auf 1 mol an Triazolidinthion der Formel (IV) im allgemeinen einen Überschuß an Oxidationsmittel ein. Setzt man Schwefel oder Kaliumsuperoxid als Oxidationsmittel ein, so verwendet man auf 1 mol an Triazolidinthion der Formel (IV) vorzugsweise 1,5 bis 2 mol an Oxidationsmittel. Setzt man Sauerstoff in Form von Luft als Oxidationsmittel ein, so leitet man einen Luftstrom vorzugsweise so lange durch das Reaktionsgemisch, bis die Umsetzung beendet ist. Die Aufarbeitung erfolgt jeweils nach üblichen Methoden. Im allgemeinen verfährt man in der Weise, daß man das Reaktionsgemisch, gegebenenfalls nach vorherigem Verdünnen mit einem mit Wasser wenig mischbaren organischen Solvens, mit wäßriger Salzlösung ausschüttelt, die organische Phase trocknet und einengt. Gegebenenfalls dann noch vorhandene Verunreinigungen lassen sich nach üblichen Methoden, wie Umkristallisation oder Chromatographie, abtrennen. Es ist jedoch auch möglich, das Produkt als Kaliumsalz zu isolieren und dann durch Zugabe von Säure freizusetzen.

Auch bei der Durchführung der zweiten Stufe des erfindungsgemäßen Verfahrens nach Variante (β) können die Reaktionstemperaturen innerhalb eines bestimmten Bereiches variiert werden. Im allgemeinen arbeitet man bei Temperaturen zwischen 50°C und 100°C.

Bei der Durchführung der zweiten Stufe des erfindungsgemäßen Verfahrens nach Variante (β) fungiert Ameisensäure sowohl als Reaktionskomponente als auch als Verdünnungsmittels. Daher setzt man auf 1 mol an Triazolidinthion der Formel (IV) im allgemeinen einen hohen Überschuß an Ameisensäure ein. Die Aufarbeitung erfolgt wiederum nach üblichen Methoden. Im allgemeinen verfährt man in der Weise, daß man das Reaktionsgemisch unter vermindertem Druck einengt und das gewünschte Produkt aus dem verbleibenden Rückstand durch Umkristallisation und/oder auf chromatographischem Wege isoliert.

In einer besonderen Variante läßt sich das erfindungsgemäße Verfahren in der Weise durchführen, daß man 1-Chlor-2-hydroxy-ethan-Derivate der Formel (VI) mit Hydrazinhydrat umsetzt und die dabei entstehenden Hydrazin-Derivate der Formel (II) dann ohne vorherige Isolierung weiter umsetzt. Demgemäß lassen sich Triazolinthione der Formel (I) auch dadurch herstellen, daß man
- 1-Chlor-2-hydroxy-ethan-Derivate der Formel 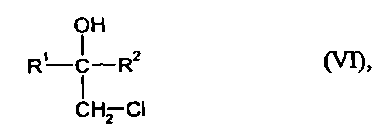
   in welcher
   - R¹ und R²: die oben angegebenen Bedeutungen haben,

   mit Hydrazinhydrat gegebenenfalls in Gegenwart eines Verdünnungsmittels umsetzt,
- die dabei erhaltenen Hydrazin-Derivate der Formel 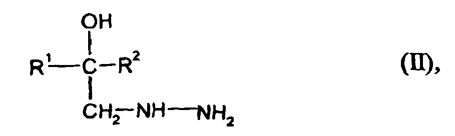
   in welcher
   - R¹ und R²: die oben angegebenen Bedeutungen haben,

   ohne vorherige Isolierung mit Formaldehyd und Thiocyanat der Formel

   X-SCN (III),

   in welcher
   - X: für Natrium, Kalium oder Ammonium steht,

   in Gegenwart eines Verdünnungsmittels und gegebenenfalls in Gegenwart einer Säure umsetzt und die entstandenen Triazolidinthione der Formel 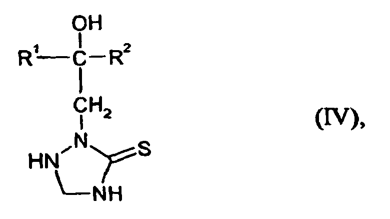
   in welcher
   - R¹ und R²: die oben angegebenen Bedeutungen haben,

   entweder
   α) mit Oxidationsmitteln gegebenenfalls in Gegenwart eines Katalysators und in Gegenwart eines Verdünnungsmittels umsetzt oder
   β) mit Ameisensäure der Formel

      HCOOH (V)

   umsetzt.

Als Verdünnungsmittel kommen bei der Durchführung der ersten Stufe dieser Variante des erfindungsgemäßen Verfahrens alle üblichen inerten organischen Solventien in Frage. Vorzugsweise verwendbar sind Alkohole, wie n-Butanol, und Ether, wie Dioxan oder Methyl-tert.-butylether und auch Nitrile, wie Acetonitril. Es ist aber auch möglich, ohne zusätzliches Lösungsmittel zu arbeiten. In diesem Fall setzt man Hydrazinhydrat im Überschuß ein, so daß es sowohl als Reaktionskomponente als auch als Verdünnungsmittel fungiert.

Die Reaktionstemperaturen können bei der Durchführung der ersten Stufe dieser Variante des erfindungsgemäßen Verfahrens in einem bestimmten Bereich variiert werden. Im allgemeinen arbeitet man bei Temperaturen zwischen 60°C und 120°C, vorzugsweise zwischen 70°C und 100°C.

Bevorzugt arbeitet man bei der Durchführung der ersten Stufe dieser Variante des erfindungsgemäßen Verfahrens in der Weise, daß man auf 1 mol an 1-Chlor-2-hydroxy-ethan-Derivat der Formel (VI) zwischen 3 und 20 mol, besonders bevorzugt zwischen 4 und 15 mol an Hydrazinhydrat einsetzt, danach mit einem mit Wasser wenig mischbaren organischen Solvens, wie Methyl-tert.-butylether, verdünnt, die wäßrige Phase abtrennt, die organische Phase wäscht und trocknet und dann ohne weitere Aufarbeitung für die folgenden Umsetzungen verwendet.

Bei der Durchführung der weiteren Stufen dieser Variante des erfindungsgemäßen Verfahrens geht man in der oben beschriebenen Weise vor.

Die erfindungsgemäß herstellbaren Triazolinthion-Derivate können in der "Thiono"-Form der Formel 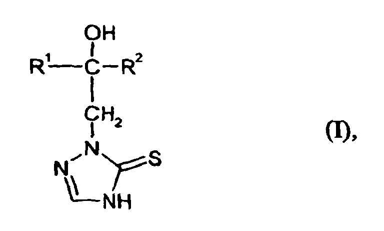 oder in der tautomeren "Mercapto"-Form der Formel 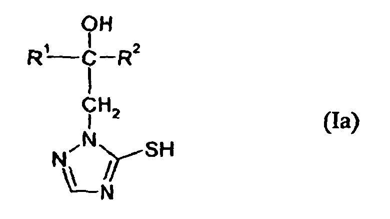 vorliegen.

Der Einfachheit halber wird jeweils nur die "Thiono"-Form aufgeführt.

Die erfindungsgemäß herstellbaren Triazolinthion-Derivate sind als Wirkstoffe mit mikrobiziden, insbesondere fungiziden Eigenschaften bekannt (vgl. WO-A 96-16048).

Die Durchführung des erfindungsgemäßen Verfahrens wird durch die folgenden Beispiele veranschaulicht.

### Herstellungsbeispiele

### Beispiel 1

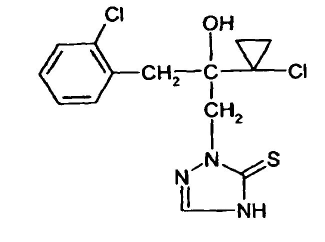

### a) Herstellung der Verbindung der Formel

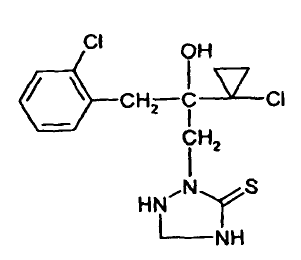

Ein Gemisch aus 5,48 g (20 mmol) [2-(1-Chlor-cycloprop-1-yl)-3-(2-chlorphenyl)-2-hydroxy]-propyl-1-hydrazin, 40 ml Methyl-tert.-butylether, 0,9 g (30 mmol) Paraformaldehyd und 1,84 g (24 mmol) Ammoniumthiocyanat wird 3 Stunden unter Rühren auf 60°C erhitzt. Nach dem Abkühlen auf Raumtemperatur wird das Reaktionsgemisch mit Methyl-tert.-butylether verdünnt und mit gesättigter, wäßriger Natriumcarbonat-Lösung gewaschen. Die organische Phase wird über Natriumsulfat getrocknet, filtriert und unter vermindertem Druck eingeengt. Man erhält auf diese Weise 6,1 g eines Produktes, das gemäß HPLC-Analyse zu 86,9 % aus 2-(1-Chlorcycloprop-1-yl)-1-(2-chlorphenyl)-2-hydroxy-3-(1,2,4-triazolidin-5-thiono-1-yl)-propan besteht. Nach Zugabe von wenig Dichlormethan fällt 2-(1-Chlor-cycloprop-1-yl)-1-(2-chlor-phenyl)-2-hydroxy-3-(1,2,4-triazolidin-S-thiono-1-yl)-propan in Form einer kristallinen Festsubstanz aus.
Schmelzpunkt: 152-154°C
¹H-NMR-Spektrum (CDCl₃, TMS, 250 MHz): δ = 0,8-1,3 (m,3H); 3,1 (d, J = 14 Hz, 1H); 3,65 (d, J = 14 Hz, 1H); 4,2 (s,2H); 4,45-4,65 (m,3H); 5,1 (t, NH); 7,15-7,6 (m,4H) ppm

### b) Herstellung der Verbindung der Formel

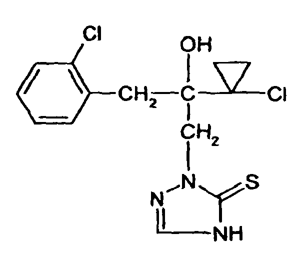

Über ein auf 70°C erwärmtes Gemisch aus 1,72 g (5 mmol) 2-(1-Chlor-cycloprop-1-yl)-1-(2-chlor-phenyl)-2-hydroxy-3-(1,2,4-triazolidin-5-thiono-1-yl)-propan, 10 ml absolutem Toluol, 0,34 g (6 mmol) Kaliumhydroxid-Pulver und 10 mg Schwefel-Pulver wird unter Rühren 3,5 Stunden lang ein Luftstrom geleitet. Dabei wird der Fortgang der Umsetzung durch HPLC-Analyse kontrolliert. Nach dem Abkühlen auf Raumtemperatur wird das Reaktionsgemisch mit Methyl-tert.-butylether verdünnt und mehrfach mit gesättigter, wäßriger Ammoniumchlorid-Lösung gewaschen. Die organische Phase wird über Natriumsulfat getrocknet, filtriert und unter vermindertem Druck eingeengt. Man erhält auf diese Weise 2,2 g eines Produktes, das gemäß HPLC-Analyse zu 71 % aus 2-(1-Chlor-cycloprop-1-yl)-1-(2-chlorphenyl)-3-(4,5-dihydro-1,2,4-triazol-5-thiono-1-yl)-propan-2-ol besteht. Nach Umkristallisation aus Toluol weist die Substanz einen Schmelzpunkt von 136°C bis 138°C auf.

### Beispiel 2

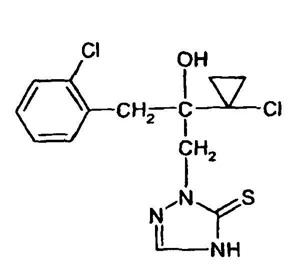

### a) Herstellung der Verbindung der Formel

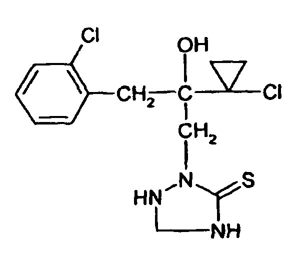

Ein Gemisch aus 2,78 g (10 mmol) 3-Chlor-2-(1-Chlor-cycloprop-1-yl)-1-(2-chlorphenyl)-propan-2-ol und 4,9 ml (100 mmol) Hydrazinhydrat wird 3 Stunden unter Rühren auf 80°C erhitzt. Anschließend kühlt man das Reaktionsgemisch auf 20°C ab, setzt 15 ml Methyl-tert.-butylether hinzu und trennt die wäßrige Phase ab. Die organische Phase wird einmal mit 5 ml Wasser gewaschen und dann über 1 g Natriumsulfat getrocknet. Danach wird das Reaktionsgemisch filtriert, mit 0,3 g (10 mmol) Paraformaldehyd und 0,76 g (20 mmol) Ammoniumthiocyanat versetzt und 3 Stunden unter Rühren auf 55 bis 60°C erhitzt. Man läßt auf Raumtemperatur abkühlen, versetzt mit Methyl-tert.-butylether und wäscht dreimal mit gesättigter, wäßriger Ammoniumchlorid-Lösung. Die organische Phase wird über Natriumsulfat getrocknet, filtriert und unter vermindertem Druck eingeengt. Man erhält auf diese Weise 2,4 g eines Produktes, das gemäß HPLC-Analyse zu 68,3 % aus 2-(1-Chlorcycloprop-1-yl)-1-(2-chlor-phenyl)-2-hydroxy-3-(1,2,4-triazolidin-5-thiono-1-yl)-propan besteht. Nach Zugabe von wenig Dichlormethan fällt 2-(1-Chlor-cycloprop-1-yl)-1-(2-chlor-phenyl)-2-hydroxy-3-(1,2,4-triazolidin-5-thiono-1-yl)-propan in Form einer kristallinen Festsubstanz aus.
Schmelzpunkt: 152-154°C

### b) Herstellung der Verbindung der Formel

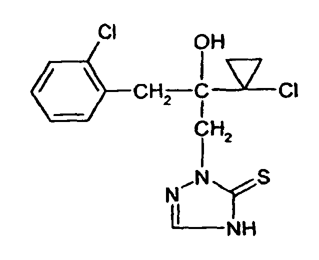

Ein Gemisch aus 1,71 g (5 mmol) 2-(1-Chlor-cycloprop-1-yl)-1-(2-chlor-phenyl)-2-hydroxy-3-(1,2,4-triazolidin-5-thiono-1-yl)-propan, 12,5 ml n-Butylacetat und 0,25 g (7,5 mmol) Schwefelpulver wird 9 Stunden unter Rühren auf 100°C erhitzt, wobei das Fortschreiten der Umsetzung durch HPLC-Analyse kontrolliert wird. Anschließend läßt man das Reaktionsgemisch auf Raumtemperautr abkühlen, verdünnt mit Methyl-tert.-butylether und wäscht mehrfach mit gesättigter, wäßriger Ammoniumchlorid-Lösung. Die organische Phase wird über Natriumsulfat getrocknet, filtriert und unter vermindertem Druck eingeengt. Man erhält auf diese Weise 2,5 g eines Produktes, daß gemäß HPLC-Analyse zu 72,2 % aus 2-(1-Chlor-cycloprop-1-yl)-1-(2-chlor-phenyl)-3-(4,5-dihydro-1,2,4-triazol-5-thiono-1-yl)-propan-2-ol besteht. Nach Umkristallisation aus Toluol weist die Substanz einen Schmelzpunkt von 137°C bis 138°C auf.

### Beispiel 3

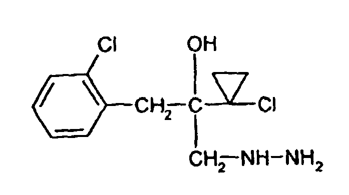

Ein Gemisch aus 27,8 g (0,1 mol) 3-Chlor-2-(1-chlor-cycloprop-1-yl)-1-(2-chlorphenyl)-propan-2-ol und 48,5 ml (1 mol) Hydrazinhydrat wird unter Stickstoffatmosphäre und unter Rühren 5 Stunden auf 100°C erhitzt. Nach dem Abkühlen des Zweiphasensystems auf Raumtemperatur wird die Hydrazin-Phase abdekantiert und der Rückstand einmal mit 20 ml Wassr gewaschen. Dabei verbleiben 25,9 g eines Produktes, daß gemäß gaschromatographischer Analyse zu 86,8 % aus 2-(1-Chlorcycloprop-1-yl)-3-(2-chlor-phenyl)-2-hydroxy-propyl-1-hydrazin besteht. Die Ausbeute errechnet sich danach zu 94,5 % der Theorie. Nach Umkristallisation des Rohproduktes aus Acetonitril erhält man 2-(1-Chlor-cycloprop-1-yl)-3-(2-chlorphenyl)-2-hydroxy-propyl-1-hydrazin in Form einer Festsubstanz vom Schmelzpunkt 86°C bis 88°C.
¹H-NMR-Spektrum (400 MHz, CDCl₃, TMS):
δ = 0,8-1,05 (m,3H); 1,25 (m,1H); 2,7 (d,1H); 2,95 (d,1H); 3,45 (d,1H); 3,6 (d,1H); 2,8-3,5 (3H,NH); 4,6 (1H,OH); 7,1-7,5 (m,4H) ppm

### Beispiel 4

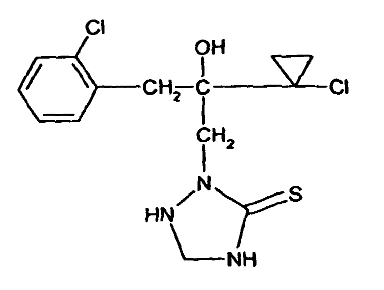

Ein Gemisch aus 2,74 g (10 mmol) aus Acetonitril umkristallisiertes [2-(1-Chlorcycloprop-1-yl)-3-(2-chlorphenyl)-2-hydroxy]-propyl-1-hydrazin, 10 ml Toluol und 0,5 ml Wasser wird bei 0°C unter Stickstoffatmosphäre vorgelegt. In dieses Gemisch leitet man unter Rühren bei 0°C Formaldehyd, der zuvor durch Depolymerisation von 0,36 g (12 mmol) Paraformaldehyd bei etwa 150°C erhalten wurde, in gasförmigem Zustand mit einem Stickstoffstrom ein. Nach beendeter Zugabe wird noch 30 Minuten bei 20°C gerührt und dann mit 0,82 g (10 mmol) Natriumthiocyanat und 1,2 g (10 mmol) Natriumhydrogensulfat versetzt. Das entstehende Reaktionsgemisch wird 2 Stunden bei 20°C gerührt und anschließend mit 100 ml Dichlormethan verdündnt. Der Feststoff wird abfiltriert, und die flüssige organische Phase wird unter verminderten Druck eingeengt. Es verbleiben 3,28 g eines Produktes, das gemäß HPLC-Analyse zu 98,6 % aus 2-(1-Chlorcycloprop-1-yl)-1-(2-chlor-phenyl)-2-hydroxy-3-(1,2,4-triazolidin-5-thiono-1-yl)-propan besteht. Die Ausbeute errechnet sich danach zu 95 % der Theorie.
Schmelzpunkt: 157-158°C.

### Beispiel 5

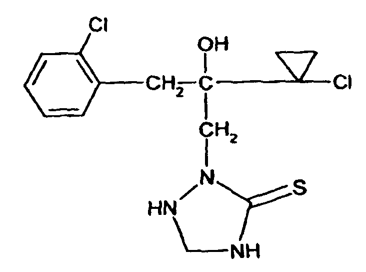

Ein Gemisch aus 2,74 g (10 mmol) aus Acetonitril umkristallisiertes [2-(1-Chlorcycloprop-1-yl)-3-(2-chlorphenyl)-2-hydrroxy]-propyl-1-hydrazin und 10 ml Toluol wird bei 0°C unter Stickstoffatmosphäre und unter Rühren mit 0,74 ml (10 mmol) einer 40 %igen Formalin-Lösung versetzt. Nach beendeter Zugabe rührt man 30 Minuten bei 20°C und versetzt dann mit 0,82 g (10 mmol) Natriumthiocyanat und 1,2 g (10 mmol) Natriumhydrogensulfat. Das Reaktionsgemisch wird 2 Stunden bei 20°C gerührt und anschließend mit 100 ml Dichlormethan verdünnt. Der Feststoff wird abfiltriert, und die flüssige organische Phase wird unter vermindertem Druck eingeengt. Es verbleiben 3,3 g eines Produktes, das gemäß HPLC-Analyse zu 93,9 % aus 2-(1-Chlor-cycloprop-1-yl)-1-(2-chlorphenyl)-2-hydroxy-3-(1,2,4-triazolidin-5-thiono-1-yl)-propan besteht. Die Ausbeute errechnet sich danach zu 90,2 % der Theorie.
Schmelzpunkt: 154-155°C.

### Beispiel 6

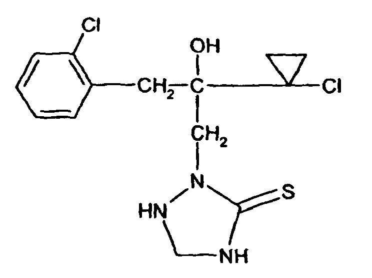

Ein Gemisch aus 2,74 g (10 mmol) aus Acetonitril umkristallisiertes [2-(1-Chlorcycloprop-1-yl)-3-(2-chlorphenyl)-2-hyxroxy]-propyl-1-hydrazin und 10 ml Toluol wird bei 0°C unter Stickstoffatmosphäre und unter Rühren nacheinander mit 1,2 g (10 mmol) Natriumhydrogensulfat, 0,74 ml (10 mmol) einer 40 %igen Formalin-Lösung und 0,82 g (10 mmol) Natriumthiocyanat versetzt. Nach beendeter Zugabe rührt man 2 Stunden bei 20°C und verdünnt dann mit 100 ml Dichlormethan. Der Feststoff wird abfiltriert, und die flüssige organische Phase wird unter vermindertem Druck eingeengt. Es verbleiben 3,4 eines Produktes, dasgemäß HPLC-Analyse zu 83,1 % aus 2-(1-Chlor-cycloprop-1-yl)-1-(2-chlorphenyl)-2-hydroxy-3-(1,2,4-triazolidin-5-thiono-1-yl)-propan besteht. Die Ausbeute erreichnet sich danach zu 83 % der Theorie.
Schmelzpunkt: 136-138°C.

### Vergleichsbeispiele

### Beispiel A

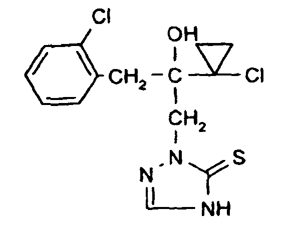

Ein Gemisch aus 3,12 g (10 mMol) 2-(1-Chlor-cycloprop-1-yl)-1-(2-chlorphenyl)-3-(1,2,4-triazol-1-yl)-propan-2-ol und 45 ml absolutem Tetrahydrofuran wird bei -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf - 10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g eines Produktes, das gemäß gaschromatographischer Analyse zu 95 % aus 2-(1-Chlorcycloprop-1-yl)-1-(2-chlor-phenyl)-3-(4,5-dihydro-1,2,4-triazol-5-thiono-1-yl)-propan-3-ol besteht. Nach Umkristallisation aus Toluol weist die Substanz einen Schmelzpunkt von 138°C bis 139°C auf.

### Beispiel B

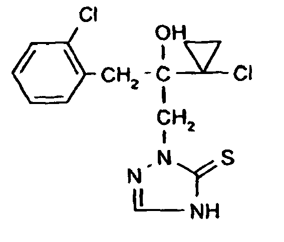

Ein Gemisch aus 3,12 g (10 mmol) 2-(1-Chlor-cycloprop-1-yl)-1-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cycloprop-1-yl)-1-(2-chlor-phenyl)-3-(4,5-dihydro-1,2,4-triazol-5-thiono-1-yl)-propan-2-ol in Form einer Festsubstanz, die bei 138-139°C schmilzt.

## Patentansprüche

1. Verfahren zur Herstellung von Triazolinthion-Derivaten der Formel 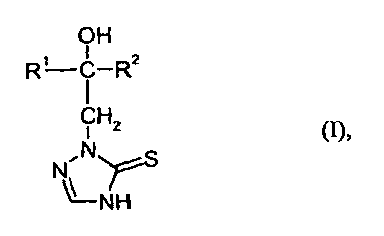 in welcher
R¹ und R² gleich oder verschieden sind und für geradkettiges oder verzweigtes Alkyl mit 1 bis 6 Kohtenstoffatomen steht, wobei diese Reste einfach bis vierfach, gleichartig oder verschieden substituiert sein können durch Halogen, Alkoxy mit 1 bis 4 Kohlenstoffatomen, Alkoximino mit 1 bis 4 Kohlenstoffatomen im Alkoxyteil und/oder Cycloalkyl mit 3 bis 7 Kohlenstoffatomen,
oder
für geradkettiges oder verzweigtes Alkenyl mit 2 bis 6 Kohlenstoffatomen steht, wobei jeder dieser Reste einfach bis dreifach, gleichartig oder verschieden substituiert sein kann durch Halogen, Alkoxy mit 1 bis 4 Kohlenstoffatomen und/oder Cycloalkyl mit 3 bis 7 Kohlenstoffatomen,
oder
für Cycloalkyl mit 3 bis 7 Kohlenstoffatomen steht, wobei jeder dieser Reste einfach bis dreifach, gleichartig oder verschieden substituiert sein kann durch Halogen, Cyano und/oder Alkyl mit 1 bis 4 Kohlenstoffatomen,
oder
für Aralkyl mit 6 bis 10 Kohlenstoffatomen im Arylteil und 1 bis 4 Kohlenstoffatomen im geradkettigen oder verzweigten Alkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoallcyl mit 1 bis 4 Kohlenstoffatomen im Alkoxyteil und 1 bis 4 Kohlenstoffatomen im Alkylteil, Nitro und/oder Cyano,
oder
für Aralkenyl mit 6 bis 10 Kohlenstoffatomen im Arylteil und 2 bis 4 Kohlenstoffatomen im Alkenylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil unt 1 bis 4 Kohlenstoffatomen im Alkylteil, Nitro und/oder Cyano,
oder
für Aroxyalkyl mit 6 bis 10 Kohlenstoffatomen im Arylteil und 1 bis 4 Kohlenstoffatomen im geradkettigen oder verzweigten Oxyalkylteil steht, wobei der Arylteil jeweils einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für Aryl mit 6 bis 10 Kohlenstoffatomen steht, wobei jeder dieser Reste einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
oder
für einen gegebenenfalls benzanellierten fünf- oder sechsgliedrigen heteroaromatischen Rest mit 1 bis 3 Heteroatomen, wie Stickstoff, Schwefel und/oder Sauerstoff steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
**dadurch gekennzeichnet, dass** man
a) in einer ersten Stufe Hydrazin-Derivate der Formel 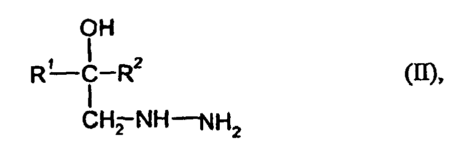
in welcher
R¹ und R² die oben angegebenen Bedeutungen haben,
mit Formaldehyd und Thiocyanat der Formel
X-SCN (III),
in welcher
X für Natrium, Kalium oder Ammonium steht,
in Gegenwart eines Verdünnungsmittels und gegebenenfalls in Gegenwart einer Säure umsetzt und
b) die entstandenen Triazolidintluione der Formel 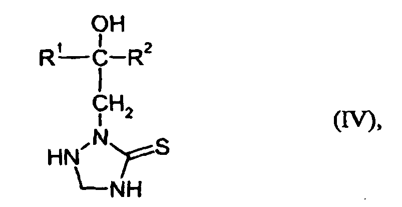
in welcher
R¹ und R² die oben angegebenen Bedeutungen haben,
in einer zweiten Stufe
entweder
α) mit Oxidationsmitteln gegebenenfalls in Gegenwart eines Katalysators und in Gegenwart eines Verdünnungsmittels umsetzt
oder
β) mit Ameisensäure der Formel
HCOOH (V)
umsetzt.

2. Verfahren gemäß Anspruch 1, **dadurch gekennzeichnet, daß** man 2-(1-Chlorcycloprop-1-yl)-3-(2-chlor-phenyl)-2-hydroxy-propyl-1-hydrazin der Formel 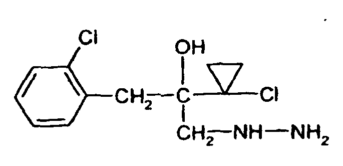
als Ausgangssubstanz einsetzt.

3. Verfahren gemäß Anspruch 1, **dadurch gekennzeichnet, daß** man Formaldehyd bei der Durchführung der ersten Stufe als Paraformaldehyd, als gasförmigen Formaldehyd oder als Formalin-Lösung einsetzt.

4. Verfahren gemäß Anspruch 1, **dadurch gekennzeichnet, daß** man bei der Durchführung der ersten Stufe Ammoniumthiocyanat als Reaktionskomponente einsetzt.

5. Verfahren gemäß Anspruch 1, **dadurch gekennzeichnet, daß** man bei der Durchführung der ersten Stufe Natriumthiocyanat in Gegenwart von einer Säure als Reaktionskomponenten einsetzt.

6. Verfahren gemäß Anspruch 1, **dadurch gekennzeichnet, daß** man bei der Durchführung der ersten Stufe Natriumthiocyanat in Gegenwart von Natriumhydrogensulfat und Wasser als Reaktionskomponenten einsetzt.

7. Verfahren gemäß Anspruch 1, **dadurch gekennzeichnet, daß** man bei der Durchführung der ersten Stufe bei Temperaturen zwischen -20°C und +100°C arbeitet.

8. Verfahren gemäß Anspruch 1, **dadurch gekennzeichnet, daß** man bei der Durchführung der zweiten Stufe nach Variante (α) bei Temperaturen zwischen 0°C und 120°C arbeitet.

9. Verfahren gemäß Anspruch 1, **dadurch gekennzeichnet, daß** man bei der Durchführung der zweiten Stufe nach Variante (β) bei Temperaturen zwischen 50°C und 100°C arbeitet.

10. Triazolidinthione der Formel 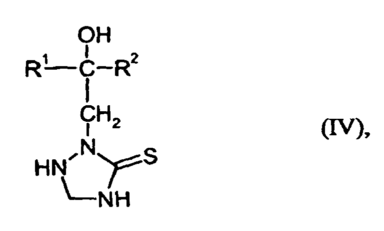 in welcher
R¹ und R² die in der Formel (I) in Anspruch 1 angegebenen Bedeutungen haben.

11. Verfahren zur Herstellung von Triazolinthion-Derivaten der Formel 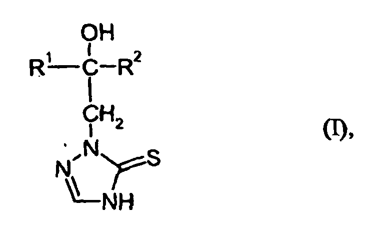 in welcher
R¹ und R² die in Formel (I) in Anspruch 1 angegebenen Bedeutungen haben,
**dadurch gekennzeichnet, dass** man
- 1-Chlor-2-hydroxy-ethan-Derivate der Formel 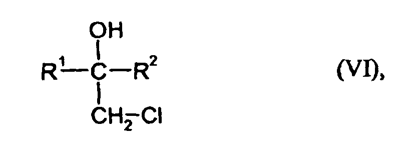
in welcher
R¹ und R² die oben angegebenen Bedeutungen haben,
mit Hydrazinhydrat gegebenenfalls in Gegenwart eines Verdünnungsmittels umsetzt,
- die dabei erhaltenen Hydrazin-Derivate der Formel 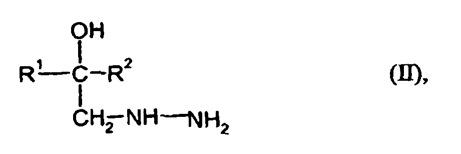
in welcher
R¹ und R² die oben angegebenen Bedeutungen haben,
ohne vorherige Isolierung mit Formaldehyd und Thiocyanat der Formel
X-SCN (III),
in welcher
X für Natrium, Kalium oder Ammonium steht,
in Gegenwart eines Verdünnungsmittels und gegebenenfalls in Gegenwart einer Säure umsetzt und
die entstandenen Triazolidinthione der Formel 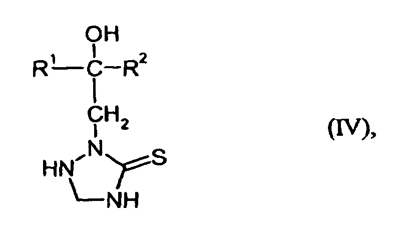
in welcher
R¹ und R² die oben angegebenen Bedeutungen haben,
entweder
α) mit Oxidationsmitteln gegebenenfalls in Gegenwart eines Katalysators und in Gegenwart eines Verdünnungsmittels umsetzt oder
β) mit Ameisensäure der Formel
HCOOH (V)
umsetzt.

## Claims

1. Process for preparing triazolinethione derivatives of the formula 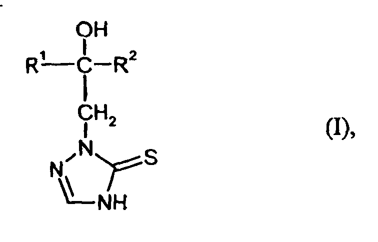
in which
R¹ and R² are identical or different and each represents straight-chain or branched alkyl having 1 to 6 carbon atoms, where these radicals may be mono- to tetrasubstituted by identical or different substituents from the group consisting of halogen, alkoxy having 1 to 4 carbon atoms, alkoximino having 1 to 4 carbon atoms in the alkoxy moiety and cycloalkyl having 3 to 7 carbon atoms,
or
represents straight-chain or branched alkenyl having 2 to 6 carbon atoms, where each of these radicals may be mono- to trisubstituted by identical or different substituents from the group consisting of halogen, alkoxy having 1 to 4 carbon atoms and cycloalkyl having 3 to 7 carbon atoms,
or
represents cycloalkyl having 3 to 7 carbon atoms, where each of these radicals may be mono- to trisubstituted by identical or different substituents from the group consisting of halogen, cyano and alkyl having 1 to 4 carbon atoms,
or
represents aralkyl having 6 to 10 carbon atoms in the aryl moiety and 1 to 4 carbon atoms in the straight-chain or branched alkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aralkenyl having 6 to 10 carbon atoms in the aryl moiety and 2 to 4 carbon atoms in the alkenyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aroxyalkyl having 6 to 10 carbon atoms in the aryl moiety and 1 to 4 carbon atoms in the straight-chain or branched oxyalkyl moiety, where the aryl moiety may in each case be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents aryl having 6 to 10 carbon atoms, where each of these radicals may be mono- to trisubstituted by identical or different substituents from the group consisting of halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy moiety, alkoximinoalkyl having 1 to 4 carbon atoms in the alkoxy moiety and 1 to 4 carbon atoms in the alkyl moiety, nitro and cyano,
or
represents an optionally benzo-fused five- or six-membered heteroaromatic radical having 1 to 3 heteroatoms, such as nitrogen, sulphur and/or oxygen, where each of these radicals may be mono- to trisubstituted by identical or different substituents from the group consisting of halogen, alkyl having 1 to 4 carbon atoms, hydroxyalkyl having 1 to 4 carbon atoms, hydroxyalkinyl having 3 to 8 carbon atoms, alkoxy having 1 or 2 carbon atoms, alkylthio having 1 or 2 carbon atoms, halogenoalkyl, halogenoalkoxy and halogenoalkylthio having in each case 1 or 2 carbon atoms and 1 to 5 identical or different halogen atoms, such as fluorine or chlorine atoms, formyl, dialkoxymethyl having 1 or 2 carbon atoms in each alkoxy group, acyl having 2 to 4 carbon atoms, alkoxycarbonyl having 1 to 4 carbon atoms in the alkoxy moiety, alkoximinoalkyl having 1 to 4 carbon atoms in the alkoxy moiety and 1 to 3 carbon atoms in the alkyl moiety, nitro and cyano,
**characterized in that**
a) in a first step hydrazine derivatives of the formula 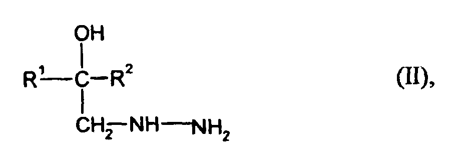
in which
R¹ and R² are each as defined above
are reacted with formaldehyde and thiocyanate of the formula
X-SCN (III),
in which
X represents sodium, potassium or ammonium,
in the presence of a diluent and if appropriate in the presence of an acid, and
b) the resulting triazolidinethiones of the formula 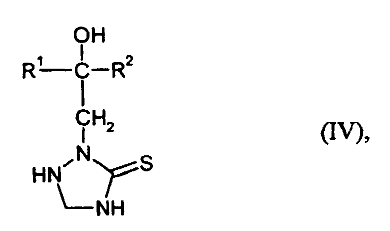
in which
R¹ and R² are each as defined above
are, in a second step,
either
α) reacted with oxidizing agents, if appropriate in the presence of a catalyst and in the presence of a diluent,
or
β) reacted with formic acid of the formula
HCOOH (V).

2. Process according to Claim 1, **characterized in that** the starting material used is 2-(1-chloro-cycloprop-1-yl)-3-(2-chloro-phenyl)-2-hydroxy-propyl-1-hydrazine of the formula

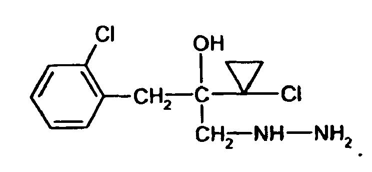

3. Process according to Claim 1, **characterized in that** the formaldehyde used for carrying out the first step is employed as paraformaldehyde, as gaseous formaldehyde or as formalin solution.

4. Process according to Claim 1, **characterized in that** the reaction component used for carrying out the first step is ammonium thiocyanate.

5. Process according to Claim 1, **characterized in that** the reaction components used for carrying out the first step are sodium thiocyanate in the presence of an acid.

6. Process according to Claim 1, **characterized in that** the reaction components used for carrying out the first step are sodium thiocyanate in the presence of sodium hydrogen sulphate and water.

7. Process according to Claim 1, **characterized in that** the first step is carried out at temperatures between -20°C and +100°C.

8. Process according to Claim 1, **characterized in that** the second step according to variant (α) is carried out at temperatures between 0°C and 120°C.

9. Process according to Claim 1, **characterized in that** the second step according to variant (β) is carried out at temperatures between 50°C and 100°C.

10. Triazolidinethiones of the formula 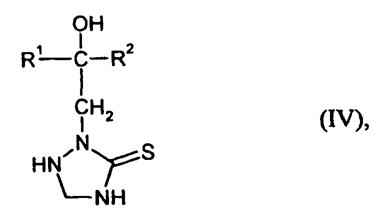
in which
R¹ and R² have the meanings given in the formula (I) in Claim 1.

11. Process for preparing triazolinethione derivatives of the formula 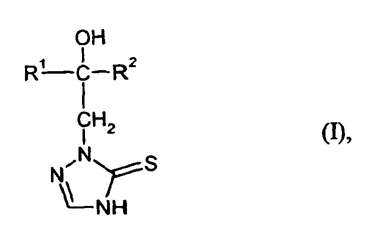
in which
R¹ and R² have the meanings given in the formula (I) in Claim 1,
**characterized in that**
- 1-chloro-2-hydroxy-ethane derivatives of the formula 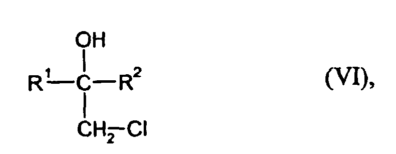
in which
R¹ and R² are each as defined above
are reacted with hydrazine hydrate, if appropriate in the presence of a diluent,
- the resulting hydrazine derivatives of the formula 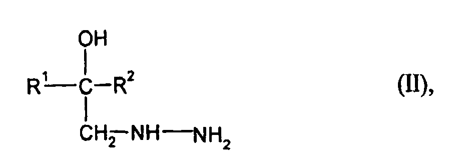
in which
R¹ and R² are each as defined above
are reacted without prior isolation with formaldehyde and thiocyanate of the formula
X-SCN (III),
in which
X represents sodium, potassium or ammonium,
in the presence of a diluent and if appropriate in the presence of an acid, and
the resulting triazolidinethiones of the formula 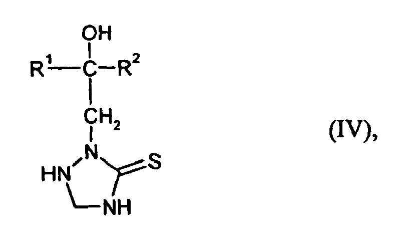
in which
R¹ and R² are each as defined above
are either
α) reacted with oxidizing agents, if appropriate in the presence of a catalyst and in the presence of a diluent, or
β) are reacted with formic acid of the formula
HCOOH (V).

## Revendications

1. Procédé de fabrication de dérivés de triazolinethione de la formule 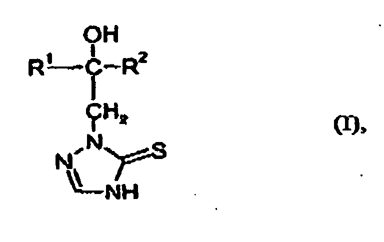
dans laquelle
R¹ et R² sont identiques ou différents et représentent un groupe alkyle à chaîne linéaire ou ramifiée avec 1 à 6 atomes de carbone, ces restes pouvant être substitués de une à quatre fois de manière identique ou différente par un halogène, un groupe alcoxy avec 1 à 4 atomes de carbone, un groupe alcoxyimino avec 1 à 4 atomes de carbone dans la partie alcoxy et/ou un groupe cycloalkyle avec 3 à 7 atomes de carbone,
ou
représentent un groupe alcényle à chaîne linéaire ou ramifiée avec 2 à 6 atomes de carbone, chacun de ces restes pouvant être substitué de une à trois fois de manière identique ou différente par un halogène, un groupe alcoxy avec 1 à 4 atomes de carbone et/ou un groupe cycloalkyle avec 3 à 7 atomes de carbone,
ou
représentent un groupe cycloalkyle avec 3 à 7 atomes de carbone, chacun de ces restes pouvant être substitué de une à trois fois de manière identique ou différente par un halogène, un groupe cyano et/ou un groupe alkyle avec 1 à 4 atomes de carbone,
ou
représentent un groupe aralkyle avec 6 à 10 atomes de carbone dans la partie aryle et 1 à 4 atomes de carbone dans la partie alkyle à chaîne linéaire ou ramifiée, la partie aryle pouvant être respectivement substituée de une à trois fois de manière identique ou différente par un halogène, un groupe alkyle avec 1 à 4 atomes de carbone, un groupe alcoxy avec 1 à 4 atomes de carbone, un groupe alkylthio avec 1 à 4 atomes de carbone, un groupe halogénoalkyle avec 1 ou 2 atomes de carbone et 1 à 5 atomes halogènes identiques ou différents, un groupe halogénoalcoxy avec 1 ou 2 atomes de carbone et 1 à 5 atomes halogènes identiques ou différents, un groupe halogénoalkylthio avec 1 ou 2 atomes de carbone et 1 à 5 atomes halogènes identiques ou différents, un groupe cycloalkyle avec 3 à 7 atomes de carbone, un groupe phényle, un groupe phénoxy, un groupe alkoxycarbonyle avec 1 à 4 atomes de carbone dans la partie alcoxy, un groupe alkoxyiminoalkyle avec 1 à 4 atomes de carbone dans la partie alcoxy et 1 à 4 atomes de carbone dans la partie alkyle, un groupe nitro et/ou un groupe cyano,
ou
représentent un groupe aralcényle avec 6 à 10 atomes de carbone dans la partie aryle et 2 à 4 atomes de carbone dans la partie alcényle, la partie aryle pouvant être respectivement substituée de une à trois fois de manière identique ou différente par un halogène, un groupe alkyle avec 1 à 4 atomes de carbone, un groupe alcoxy avec 1 à 4 atomes de carbone, un groupe alkylthio avec 1 à 4 atomes de carbone, un groupe halogénoalkyle avec 1 ou 2 atomes de carbone et 1 à 5 atomes halogènes identiques ou différents, un groupe halogénoalcoxy avec 1 ou 2 atomes de carbone et 1 à 5 atomes halogènes identiques ou différents, un groupe halogénoalkylthio avec 1 ou 2 atomes de carbone et 1 à 5 atomes halogènes identiques ou différents, un groupe cycloalkyle avec 3 à 7 atomes de carbone, un groupe phényle, un groupe phénoxy, un groupe alcoxycarbonyle avec 1 à 4 atomes de carbone dans la partie alcoxy, un groupe alcoxyiminoalkyle avec 1 à 4 atomes de carbone dans la partie alcoxy et 1 à 4 atomes de carbone dans la partie alkyle, un groupe nitro et/ou un groupe cyano,
ou
représentent un groupe aroxyalkyle avec 6 à 10 atomes de carbone dans la partie aryle et 1 à 4 atomes de carbone dans la partie oxyalkyle à chaîne linéaire ou ramifiée, la partie aryle pouvant être respectivement substituée de une à trois fois de manière identique ou différente par un halogène, un groupe alkyle avec 1 à 4 atomes de carbone, un groupe alcoxy avec 1 à 4 atomes de carbone, un groupe alkylthio avec 1 à 4 atomes de carbone, un groupe halogénoalkyle avec 1 ou 2 atomes de carbone et 1 à 5 atomes halogènes identiques ou différents, un groupe halogénoalcoxy avec 1 ou 2 atomes de carbone et 1 à 5 atomes halogènes identiques ou différents, un groupe halogénoalkylthio avec 1 ou 2 atomes de carbone et 1 à 5 atomes halogènes identiques ou différents, un groupe cycloalkyle avec 3 à 7 atomes de carbone, un groupe phényle, un groupe phénoxy, un groupe alcoxycarbonyle avec 1 à 4 atomes de carbone dans la partie alcoxy, un groupe alcoxyiminoalkyle avec 1 à 4 atomes de carbone dans la partie alcoxy et 1 à 4 atomes de carbone dans la partie alkyle, un groupe nitro et/ou un groupe cyano,
ou
représentent un groupe aryle avec 6 à 10 atomes de carboné, chacun de ces restes pouvant être substitué de une à trois fois de manière identique ou différente par un halogène, un groupe alkyle avec 1 à 4 atomes de carbone, un groupe alcoxy avec 1 à 4 atomes de carbone, un groupe alkylthio avec 1 à 4 atomes de carbone, un groupe halogénoalkyle avec 1 ou 2 atomes de carbone et 1 à 5 atomes halogènes identiques ou différents, un groupe halogénoalcoxy avec 1 ou 2 atomes de carbone et 1 à 5 atomes halogènes identiques ou différents, un groupe halogénoalkylthio avec 1 ou 2 atomes de carbone et 1 à 5 atomes halogènes identiques ou différents, un groupe cycloalkyle avec 3 à 7 atomes de carbone, un groupe phényle, un groupe phénoxy, un groupe alcoxycarbonyle avec 1 à 4 atomes de carbone dans la partie alcoxy, un groupe alcoxyiminoalkyle avec 1 à 4 atomes de carbone dans la partie alcoxy et 1 à 4 atomes de carbone dans la partie alkyle, un groupe nitro et/ou un groupe cyano,
ou
représentent un reste hétéroaromatique à cinq ou six cycles, le cas échéant accolé au benzène, avec 1 à 3 hétéroatomes, tels que l'azote, le soufre et/ou l'oxygène, chacun de ces restes pouvant être substitué de une à trois fois de manière identique ou différente par un halogène, un groupe alkyle avec 1 à 4 atomes de carbone, un groupe hydroxyalkyle avec 1 à 4 atomes de carbone, un groupe hydroxyalcynyle avec 3 à 8 atomes de carbone, un groupe alcoxy avec 1 ou 2 atomes de carbone, un groupe alkylthio avec 1 ou 2 atomes de carbone, un groupe halogénoalkyle, un groupe halogénoalcoxy et un groupe halogénoalkylthio avec respectivement 1 ou 2 atomes de carbone et 1 à 5 atomes halogènes identiques ou différents, tels que des atomes de fluor ou de chlore, un groupe formyle, un groupe dialcoxyméthyle avec 1 ou 2 atomes de carbone dans chaque groupe alcoxy, un groupe acyle avec 2 à 4 atomes de carbone, un groupe alcoxycarbonyle avec 1 à 4 atomes de carbone dans la partie alcoxy, un groupe alcoxyiminoalkyle avec 1 à 4 atomes de carbone dans la partie alcoxy et 1 à 3 atomes de carbone dans la partie alkyle, un groupe nitro et/ou un groupe cyano,
**caractérisé en ce que**
a) on fait réagir dans une première étape des dérivés de l'hydrazine de la formule 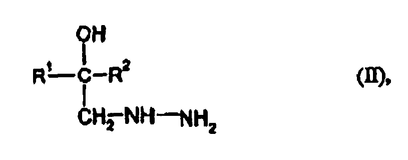
dans laquelle
R¹ et R² ont la signification donnée ci-dessus,
avec du formaldéhyde et du thiocyanate de la formule
X-SCN (III),
dans laquelle.
X représente le sodium, le potassium ou l'ammonium,
en présence d'un diluant et le cas échéant en présence d'un acide et
b) on fait réagir les triazolidinethiones formées de la formule 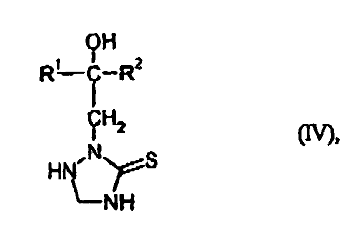
dans laquelle
R¹ et R² ont la signification donnée ci-dessus,
dans une deuxième étape
soit
α) avec des agents d'oxydation le cas échéant en présence d'un catalyseur et en présence d'un diluant
ou
β) avec de l'acide formique de la formule
HCOOH (V)

2. Procédé suivant la revendication 1, **caractérisé en ce qu'**on utilise comme substance de départ de la 2-(1-chlorocycloprop-1-yl)-3-(2-chloro-phényl)-2-hydroxy-propyl-1-hydrazine de la formule

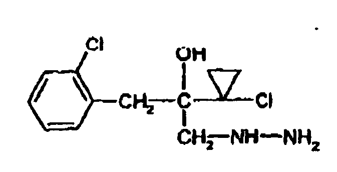

3. Procédé suivant la revendication 1, **caractérisé en ce qu'**on utilise pour la réalisation de la première étape du formaldéhyde sous la forme de paraformaldéhyde, de formaldéhyde gazeux ou de solution de formaline.

4. Procédé suivant la revendication 1, **caractérisé en ce qu'**on utilise du thiocyanate d'ammonium comme composant de la réaction pour la réalisation de la première étape.

5. Procédé suivant la revendication 1, **caractérisé en ce qu'**on utilise du thiocyanate de sodium en présence d'un acide comme composant de la réaction pour la réalisation de la première étape.

6. Procédé suivant la revendication 1, **caractérisé en ce qu'**on utilise du thiocyanate de sodium en présence d'hydrogénosulfate de sodium et d'eau comme composant de la réaction pour la réalisation de la première étape.

7. Procédé suivant la revendication 1, **caractérisé en ce qu'**on effectue la première étape à des températures comprises entre -20°C et +100°C.

8. Procédé suivant la revendication 1, **caractérisé en ce qu'**on effectue la deuxième étape suivant la version (α) à des températures comprises entre 0°C et 120°C.

9. Procédé suivant la revendication 1, **caractérisé en ce qu'**on effectue la deuxième étape suivant la version (β) à des températures comprises entre 50°C et 100°C.

10. Triazolidinethiones de la formule 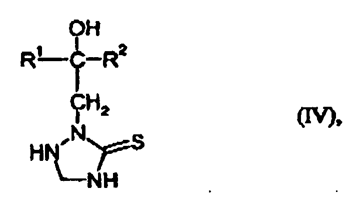
dans laquelle R¹ et R² ont les significations indiquées dans la revendication 1 pour la formule (I).

11. Procédé de fabrication de dérivés de triazolinethione de la formule 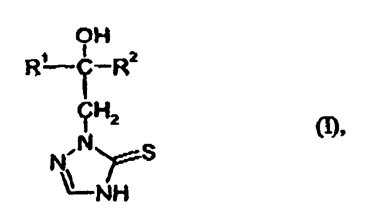
dans laquelle R¹ et R² ont les significations indiquées dans la revendication 1 pour la formule (I),
**caractérisé en ce que**
- on fait réagir des dérivés du 1-chloro-2-hydroxy-éthane de la formule 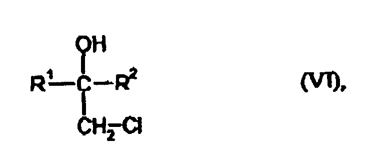
dans laquelle
R¹ et R² ont la signification donnée ci-dessus,
avec de l'hydrate d'hydrazine le cas échéant en diluant d'un diluant,
- on fait réagir les dérivés de l'hydrazine obtenus de la formule 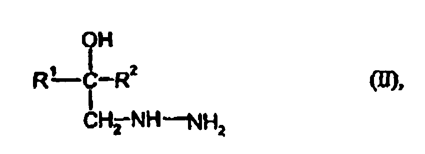
dans laquelle
R¹ et R² ont la signification donnée ci-dessus,
sans séparation préalable avec du formaldéhyde et du thiocyanate de la formule
X-SCN (III),
dans laquelle
X représente le sodium, le potassium ou l'ammonium,
en présence d'un diluant et le cas échéant en présence d'un acide et
on fait réagir les triazolidinethiones formées de la formule 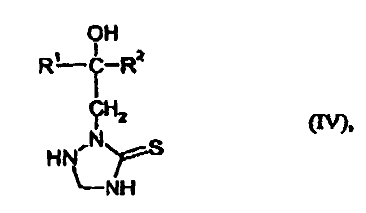
dans laquelle
R¹ et R² ont la signification donnée ci-dessus,
soit
a) avec des agents d'oxydation le cas échéant en présence d'un catalyseur et en présence d'un diluant ou
β) avec de l'acide formique de la formule
HCOOH (V)
